# EUROPÄISCHE PATENTSCHRIFT

(11) **EP 1 045 399 B1**
(45) Veröffentlichungstag und Bekanntmachung des Hinweises auf die Patenterteilung: **24.08.2005**
(21) Anmeldenummer: 99107121.8
(22) Anmeldetag: 12.04.1999
(51) Int. Cl.: G21K 5/10, A61N 5/10

(54) **Vorrichtung und Verfahren zur Regelung eines Rasterscanners in der Ionenstrahltherapie**
Device and method for controlling a raster scanner in ion theraphy
Dispositif et procédé de contrôle d'un système à balayage de trame pour thérapie ionique

(43) Veröffentlichungstag der Anmeldung: 18.10.2000
(73) Patentinhaber: GSI Gesellschaft für Schwerionenforschung mbH, 64291 Darmstadt-Wixhausen (DE)
(72) Erfinder: Haberer, Thomas, Dr., D-64291 Darmstadt (DE); Ott, Wolfgang, Dr., D-64291 Darmstadt (DE)
(74) Vertreter: Boeters, Hans Dietrich

(56) Entgegenhaltungen:
- DE-A- 3 311 786
- US-A- 5 017 789
- EICKHOFF H ET AL: "The GSI Cancer Therapy Project" PROCEEDINGS OF THE 1997 PARTICLE ACCELERATOR CONFERENCE (CAT. NO.97CH36167), PROCEEDINGS OF THE 1997 PARTICLE ACCELERATOR CONFERENCE, VANCOUVER, BC, CANADA, 12-16 MAY 1997, Seiten 3801-3803 vol.3, XP002116158 1998, Piscataway, NJ, USA, IEEE, USA ISBN: 0-7803-4376-X
- PEDRONI E ET AL: "THE 200-MEV PROTON THERAPY PROJECT AT THE PAUL SCHERRER INSTITUTE: CONCEPTUAL DESIGN AND PRACTICAL REALIZATION" MEDICAL PHYSICS, Bd. 22, Nr. 1, 1. Januar 1995 (1995-01-01), Seiten 37-53, XP000505145 ISSN: 0094-2405
- STOVER G ET AL: "A raster scanning power supply system for controlling relativistic heavy ion beams at the Bevalac biomedical facility" PROCEEDINGS OF THE 1987 IEEE PARTICLE ACCELERATOR CONFERENCE: ACCELERATOR ENGINEERING AND TECHNOLOGY (CAT. NO.87CH2387-9), WASHINGTON, DC, USA, 16-19 MARCH 1987, Seiten 1410-1412 vol.3, XP002116159 1987, New York, NY, USA, IEEE, USA

## Beschreibung

Die Erfindung betrifft eine Vorrichtung und ein Verfahren zur Regelung eines Rasterscanners in der Ionenstrahlentherapie gemäß dem Oberbegriff der Ansprüche 1 und 4.

Eine derartige Regelvorrichtung weist folgende Geräte mindestens auf:
- Scannermagnetstrom-Netzgeräte für horizontal und vertikal in bezug auf die Ionenstrahlmitte ablenkende Ionenstrahl-Scannermagnete, wobei die Netzgeräte von Steuer- und Auslesemodulen für die Scannermagnete gesteuert werden,
- einen ortsempfindlichen Detektor zur Ortsmessung, der über ein Steuer- und Auslesemodul gesteuert wird, und
- ein Ablaufsteuergerät, das den Ansteuerungs- und Ausleseablauf unter den Geräten der Vorrichtung steuert.

Ein derartiger Rasterscanner ist aus der europäischen Patentanmeldung EP 98 117 256.2 (Veröffentlichungs Nr. EP-0986070; Stand der Technik nach Art.54.3 EPÜ) bekannt. In dieser Druckschrift ist ein intensitätsgeregeltes Rasterscanverfahren beschrieben. Dieses Verfahren erlaubt es, trotz erheblicher Intensitätsschwankungen des Therapiestrahl, der aus Ionen besteht, und Intensitätsschwankungen zwischen einem Maximalwert und dem Mittelwert um einen Faktor 30 aufweist, die Vorgaben einer Bestrahlungsplanung derart präzise umzusetzen, daß die aus der Gesamtbestrahlung resultierende Dosisverteilung im Mittel um weniger als 5 % von der geplanten Dosisverteilung abweicht. Die Intensitätsregelung bewirkt folglich, daß die gesamte Strahlendosis pro Strahlposition trotz der erheblichen Intensitätsschwankungen eines Ionenstrahls sehr genau eingehalten werden kann.

Problematisch ist jedoch, die Realisierung einer geometrisch exakten Anwendung der Dosisverteilung zu erreichen, da nicht nur die Intensität des Therapiestrahls, sondern auch die Strahlposition des fokussierten Therapiestrahls während der Strahlapplikation signifikant schwankt. Für dieses Problem existiert derzeit keine vollständige und effiziente Lösung. Derzeit wird deshalb ein enormer Aufwand getrieben, um diese Positionsschwankungen für alle denkbaren Beschleuniger- und Hochenergiestrahl-Führungseinstellungen zu vermessen und Korrekturtabellen anzulegen. Dabei sind beispielsweise 255 Energiestufen mit jeweils 7 Fokussierstufen und jeweils 15 Intensitätsschritten zu berücksichtigen, so daß etwa 25.000 Kombinationen für jede Strahlposition zu vermessen sind und Korrekturtabellen für entsprechende Therapiegeräte anzulegen sind. Derartige Korrekturtabellen können dann zur Erzeugung der Steuerdaten des Systems herangezogen werden. Aber auch dieser Aufwand führt nur dann zu einem positiven Ergebnis, wenn die Abweichungen in der Strahlposition für jede Strahlposition reproduzierbar sind, wovon jedoch nicht generell ausgegangen werden kann.

Der Erfindung liegt deshalb die Aufgabe zugrunde, den Korrekturaufwand drastisch zu reduzieren und die geometrische Präzision signifikant zu erhöhen.

Gelöst wird diese Aufgabe durch den Gegenstand der Ansprüche 1 und 4. Weitere Merkmale bevorzugter Ausführungsformen werden in Unteransprüchen, die von den Ansprüchen 1 und 4 abhängig sind, beschrieben.

"The GSI Cancer Therapy Project", H.Eickhoff et al. offenbart eine Vorrichtung und ein Verfahren zur Regelung eines Rasterscanners in der Ionenstrahltherapie, wobei Vieldraht-Proportional-Kammern zur Kontrolle/Regelung der Strahlposition verwendet werden.

Eine erfindungsgemäße Vorrichtung zur Regelung eines Rasterscanners in der Ionenstrahlentherapie weist mindestens folgende Geräte auf:

Scannermagnetstrom-Netzgeräte für horizontal und vertikal in bezug auf die Ionenstrahlmitte ablenkende Ionenstrahl-Scannermagnete, wobei die Netzgeräte von Steuer- und Auslesemodulen für die Scannermagnete gesteuert werden, einen ortsempfindlichen Detektor der zur Ortsmessung über ein Steuer- und Auslesemodul gesteuert wird, ein Ablaufsteuergerät, das den Ansteuerungs- und Ausleseablauf unter den Geräten der Vorrichtung steuert, wobei die Vorrichtung weiterhin eine Schaltungsanordnung in dem Ablaufsteuergerät mit einer Rückkopplungsschleife zwischen den Steuer- und Auslesemodulen für die Scannermagnete und das Steuer- und Auslesemodul des ortsempfindlichen Detektors aufweist, und schaltungs- und ablauftechnisch die Steuer- und Auslesemodule für die Scannermagnete und das Steuer- und Auslesemodul des ortsempfindlichen Detektors in dem Ablaufsteuergerät derart angeordnet sind, daß die Steuer- und Auslesemodule für die Scannermagnete dem Steuer- und Auslesemodul des ortsempfindlichen Detektors seriell nachgeordnet sind.

Diese Vorrichtung verbessert neben der Qualität der Dosisapplikation auch den Patientendurchsatz und damit die Wirtschaftlichkeit der Vorrichtung, da die Steigerung der geometrischen Präzision die Zahl der Bestrahlungsunterbrechungen aufgrund von Interlocks des Ortsmeßsystems reduziert. Darüber hinaus profitieren von dieser Lösung nicht nur Rasterscansysteme in fixierten Strahlführungen mit beliebigem Winkel, sondern vielmehr und insbesondere auch die Rasterscantechnik in Kombination mit einer drehbaren Strahlführung, einer Gantry, bei der mit einer Zunahme der Positionsfehler zu rechnen ist. Solche Gantrysysteme sind nämlich extrem schwer und neigen deshalb zu mechanischer Deformation der Strahlführung.

Darüber hinaus ist die Ionenoptik sehr empfindlich in bezug auf Positionsschwankungen. Eine Magnetfeldhomogenität, insbesondere in dem letzten ablenkenden Dipolmagneten, ist äußerst schwierig herstellbar, so daß insgesamt für derartige Gantrysysteme die vorliegende erfindungsgemäße Vorrichtung zur Regelung eines Rasterscanners erhebliche Vorteile bringt, da der Ionen-Therapiestrahl in einer Bestrahlungsposition entsprechend einer Bestrahlungsplanung der erfindungsgemäßen Vorrichtung nachgeregelt und nachgeführt werden kann.

In einer bevorzugten Ausführungsform der Erfindung wird als ortsempfindlicher Detektor eine Vieldraht-Proportional-Kammer eingesetzt. Eine derartige Kammer hat den Vorteil, daß einerseits der Istzustand der Strahlposition in seinen Ortskoordinaten genau bestimmbar wird, und andererseits durch Einkoppeln des ortsempfindlichen Detektors in eine Regelungsschleife der Bestrahlungsort mit dem Bestrahlungsplan abgeglichen werden kann und eine exakte Übereinstimmung zwischen Ist- und Sollwert in bezug auf den Ort der Bestrahlung erreicht werden kann.

Zur gleichzeitigen Intensitätsregelung kann die Vorrichtung vorzugsweise mindestens eine Ionisationskammer aufweisen, die mit einem Steuer- und Auslesemodul zusammenwirkt. Dieser Steuer- und Auslesemodul der Ionisationskammer ist vorzugsweise innerhalb der Ablaufsteuerung schaltungs- und ablauftechnisch vor dem Steuer- und Auslesemodul des ortsempfindlichen Detektors angeordnet. Damit wird vorteilhaft erreicht, daß zunächst die Bestrahlungsdosis pro Strahlposition überwacht und eingehalte wird, indem eine Intensitätssteuerung sichergestellt wird und danach unabhängig von der Intensitätssteuerung mit Hilfe des ortsempfindlichen Detektors eine genaue Positionierung des Ionenstrahls eingeregelt werden kann.

Für das Verfahren zur Regelung eines Rasterscanners in der Ionenstrahlentherapie weist der Rasterscanner folgende Geräte auf:
- Scannermagnetstrom-Netzgeräte für horizontal und vertikal in bezug auf die Ionenstrahlmitte ablenkende Ionenstrahl-Scannermagnete, wobei die Netzgeräte von Steuer- und Auslesemodulen für die Scannermagnete gesteuert werden,
- einen ortsempfindlichen Detektor zur Ortsmessung, der über ein Steuer- und Auslesemodul gesteuert wird, und
- ein Ablaufsteuergerät, das den Ansteuerungs- und Ausleseablauf unter den Geräten der Vorrichtung steuert,
wobei folgende Verfahrensschritte durchgeführt werden:
- Vergleichen einer in dem Ortsmeßsteuer- und Auslesemodul abgelegten Information über die Sollposition der Strahlplanung mit der gemessenen Istposition der Strahlposition aus dem ortsempfindlichen Detektor in Echtzeit,
- Ermitteln eines Korrekturwertes für die Scannermagnetstrom-Netzgeräte des Rasterscanners und
- Einstellen des Korrekturwertes für die horizontalen und vertikalen Scannermagnetstrom-Netzgeräte des Rasterscanners und Nachführen der Strahlposition.

Ein Vorteil des erfindungsgemäßem Verfahrens besteht darin, daß die Vermessung einer großen Menge von Systemeinstellungen an dem Beschleuniger und/oder der Strahlführung im Rahmen der Qualitätssicherung und Vorbereitung der Bestrahlungsanlage für Patientenbestrahlungen dramatisch reduziert werden kann, indem die Ortsinformation aus dem positionsempfindlichen Detektor vor dem Patienten zur Nachführung der Strahlposition in Echtzeit während der Strahlanwendung verwendet werden kann. Darüber hinaus werden durch diese Erfindung die Anforderungen an die Reproduzierbarkeit der Strahlposition für alle Hochenergiestrahlungs-Einstellungen wesentlich erleichtert und die geometrische Präzision der Dosisanwendung verbessert.

Zur Nachführung der Strahlposition wird somit die im Ortsmeßsteuer- und Auslesemodul des Kontrollsystems abgelegte Information über die Sollposition aus der Bestrahlungsplanung mit der gemessenen Istposition aus dem ortsempfindlichen Detektor in Echtzeit verglichen und ein Korrekturwert für die Magnetnetzgeräte des Rasterscanners ermittelt und eingestellt. Diese Korrektur kann von Meßzyklus zu Meßzyklus des Ortsmeßsystems, z.B. innerhalb von 150 µs oder auch von einer Strahlposition im Bestrahlungsplan zur nächsten Strahlposition erfolgen. Innerhalb der Echtzeitsteuerung des Systems sind eine Reihe von Steuer- und Auslesemodulen über Schnittstellen miteinander verbunden. Für die Erfindung sind jedoch die beiden Steuer- und Auslesemodule relevant, die den Ortsmeßdetektor und die beiden Rasterscanner-Magnetstrom-Netzgeräte steuern und auslesen.

Für jeden Meßzyklus errechnet die Echtzeit-Software in dem Steuer- und Auslesemodul des ortsempfindlichen Detektors den Istwert der Strahlposition aus dem Detektor Rohdaten und verschickt diese Information über die Datenverbindung an die Steuer- und Auslesemodule der Scannermagnete. Für jeden Regelzyklus vergleicht die Echtzeit-Software in dem Steuer- und Auslesemodul der Scannermagnete die Soll- und Istposition und errechnet Stromkorrekturwerte für das horizontale und vertikale Magnetstrom-Netzgerät des Rasterscanners und stellt dann die korrigierten Stromwerte ein, welche zu verbesserten Magnetfeldeinstellungen in den Scannermagneten führen, wodurch die Strahlposition verbessert wird.

In einer bevorzugten Ausführungsform wird die Strahlnachführung mit einer über die Echtzeit-Software in den Steuer- und Auslesemodulen für die Steuermagnete einstellbaren Dämpfung durchgeführt. Damit werden vorteilhaft Regelschwingungen vermieden und Schleppfehler werden vermindert.

In einer weiteren bevorzugten Ausführungsform werden obere Schwellen festgelegt, um die Ortskorrektur zu begrenzen, um grob fehlerhafte Strahlpositionseinstellungen aus sicherheitstechnischen Gründen zu vermeiden. Sollte ein derartiger oberer Schwellenwert überschritten werden, wird vorzugsweise eine schnelle Strahlabschaltung von dem Steuer- und Auslösemodul des ortsempfindlichen Detektors in Echtzeit ausgelöst und damit eine Kette von Abschaltbefehlen für die unterschiedlichen Beschleuniger- und Strahlführungskomponenten ausgelöst.

Weitere Vorteile, Merkmale und Anwendungsmöglichkeiten der Erfindung werden nun anhand von Ausführungsbeispielen näher erläutert.
Fig. 1 zeigt einen Datenflußplan zur Regelung eines Rasterscanners in einer bevorzugten Ausführungsform der Erfindung.
Fig. 2 zeigt eine bevorzugte Ausführungsform der Erfindung als Blockschaltbild.
Fig. 3a zeigt einen Soll-/Istpositionsvergleich eines Ionen-Therapiestrahls eines Rasterscanners vor einem Einschalten einer erfindungsgemäßen Vorrichtung.
Fig. 3b zeigt einen Soll-/Istpositionsvergleich der Fig. 3a nach Einschalten der Vorrichtung zur Positionsregelung von Strahlposition zu Strahlposition eines Rasterscanners gemäß einer Ausführungsform der Erfindung.
Fig. 4a zeigt einen weiteren Soll-/Istpositionsvergleich eines Ionen-Therapiestrahls vor einem Einschalten einer erfindungsgemäßen Vorrichtung.
Fig. 4b zeigt einen Soll-/Istpositionsvergleich der Fig. 4a nach Einschalten der Vorrichtung zur Positionsregelung von Meßzyklus zu Meßzyklus gemäß einer weiteren Ausführungsform der Erfindung.

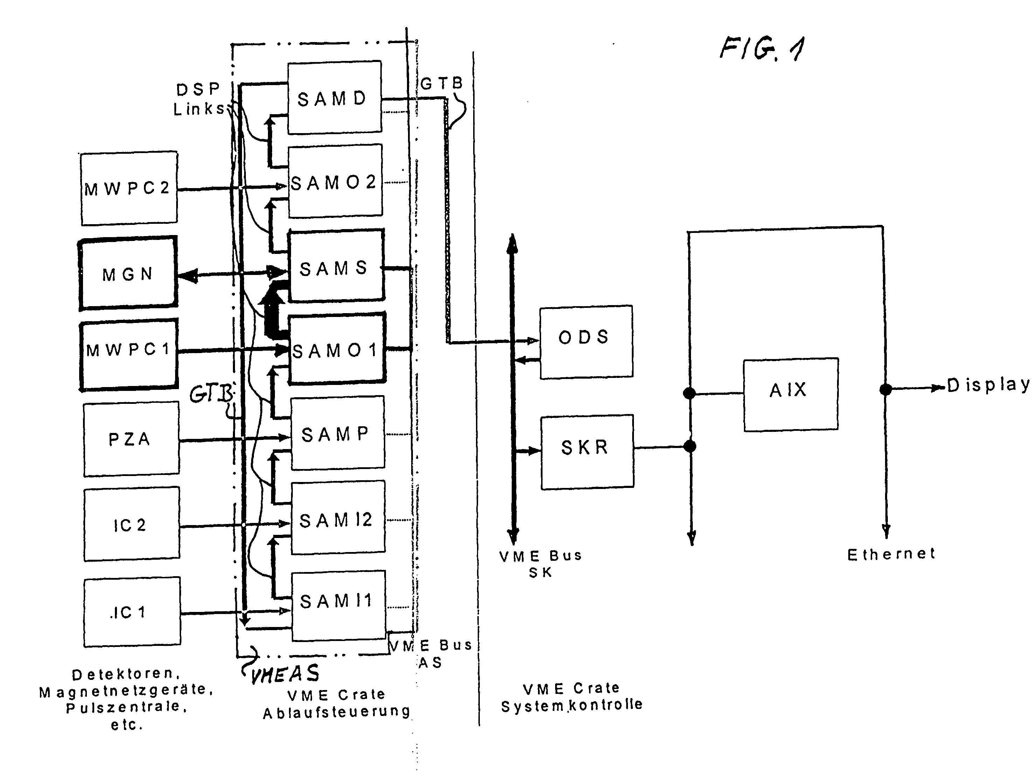

Fig. 1 zeigt einen Datenflußplan zur Regelung eines Rasterscanners in einer bevorzugten Ausführungsform der Erfindung. Dieser Datenflußplan zeigt in einer äußerst linken Spalte eine Auswahl von Geräten, die für eine Ionenstrahlentherapie eingesetzt werden, wie vorzugsweise Detektoren (IC1, IC2, MWPC1 und MWPC2), Magnetnetzgeräten (MGN) und Pulszentralenansteuerungen (PZA). In der zweiten Spalte von links wird der Datenfluß in einem Ablaufsteuergerät (VMEAS) dargestellt, das Steuer- und Auslesemodule (SAM) aufweist, die mit den Detektoren, Magnetnetzgeräten und Impulszentralenansteuerungen zusammenwirken, wobei die Steuer- und Auslesemodule (SAMI1 und SAMI2) mit Ionisationskammern (IC1 und IC2) zur Messung der Ionenstrahlteilchenzahl zusammenwirken und nach Erreichen einer durch den Behandlungsplan vorgegebenen Ionenstrahlteilchenzahl für eine Strahlposition über das Steuer- und Auslesemodul (SAMP) für die Impulszentralenansteuerung (PZA) bewirken, daß der Ionenstrahl mit Hilfe der Pulszentralenansteuerung (PZA) auf die nächste Strahlposition umgeschaltet wird.

Für die erfindungsgemäße Regelung eines Rasterscanners in der Ionenstrahlentherapie sind von den Geräten und den Steuer- und Auslesemodulen mindestens folgende Geräte erforderlich: ein ortsempfindlicher Detektor (MWPC1) zur Ortsmessung, der über ein Steuer- und Auslesemodul (SAMO1) gesteuert wird, Scannermagnetstrom-Netzgeräte (MGN) für eine horizontale (X) und eine vertikale (Y) Ablenkung des Ionen-Therapiestrahls aus der Ionenstrahlmitte mittels der Scannermagnete, wobei die Netzgeräte (MGN) von Steuer- und Auslesemodulen (SAMS) für die Scannermagnete gesteuert werden. Ein Ablaufsteuergerät (VMEAS) steuert den Ansteuerungs- und Ausleseablauf unter den für die erfindungsgemäße Vorrichtung erforderlichen Geräten.

Dazu stellt der Datenflußplan nach Fig. 1 eine Schaltungsanordnung in dem Ablaufsteuergerät (VMEAS) mit einer Rückkopplungsschleife zwischen den Steuer- und Auslesemodulen (SAMS) für die Scannermagnete und dem Steuer- und Auslesemodul (SAMO1) des ortsempfindlichen Detektors (MWPC1) dar. Für diese Regelungsschleife sind die Steuer- und Auslesemodule (SAMS) für die Steuermagnete und das Steuer- und Auslesemodul (SAMO1) des ortsempfindlichen Detektors (MWPC1) in dem Ablaufsteuergerät (VMEAS) derart schaltungs- und ablauftechnisch angeordnet, daß die Steuer- und Auslesemodule (SAMS) für die Scannermagnete dem Steuer- und Auslesemodul (SAMO1) des ortsempfindlichen Detektors (MWPC1) seriell nachgeordnet sind. Die Steuer- und Auslesemodule (SAMS) für die Scannermagnete und das Steuer- und Auslesemodul (SAMO1) des ortsempfindlichen Detektors (MWPC1) bestehen aus äußerst schnellen Mikroprozessoren, die über entsprechende Digital-Signal-Prozessor-Links (DSP-Links) untereinander kommunizieren. Die Rückkopplungsschleife zwischen den Steuer- und Auslesemodulen (SAMS) für die Scannermagnete und das Steuer- und Auslesemodul (SAMO1) des ortsempfindlichen Detektors wird einerseits durch die Digital-Signal-Prozessor-Links und einen Ablaufsteuerbus (VME-Bus-AS) gebildet.

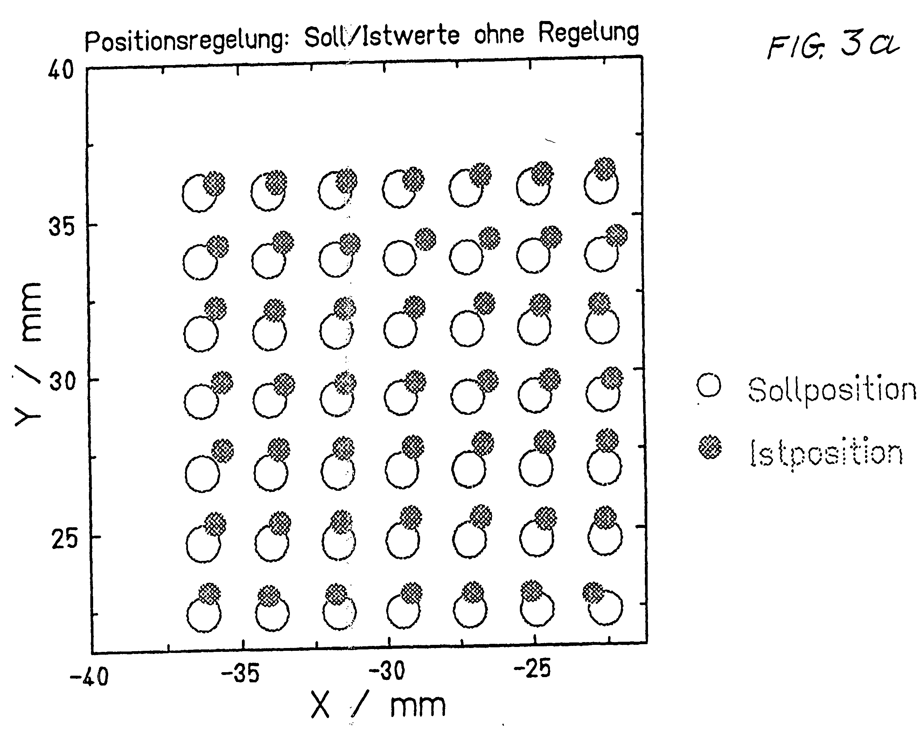

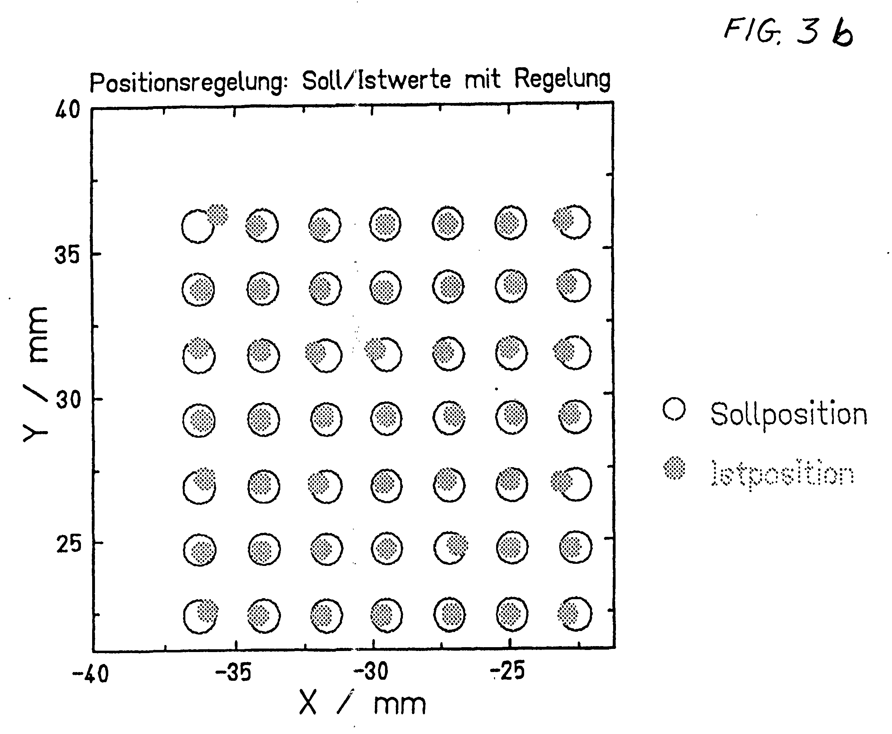

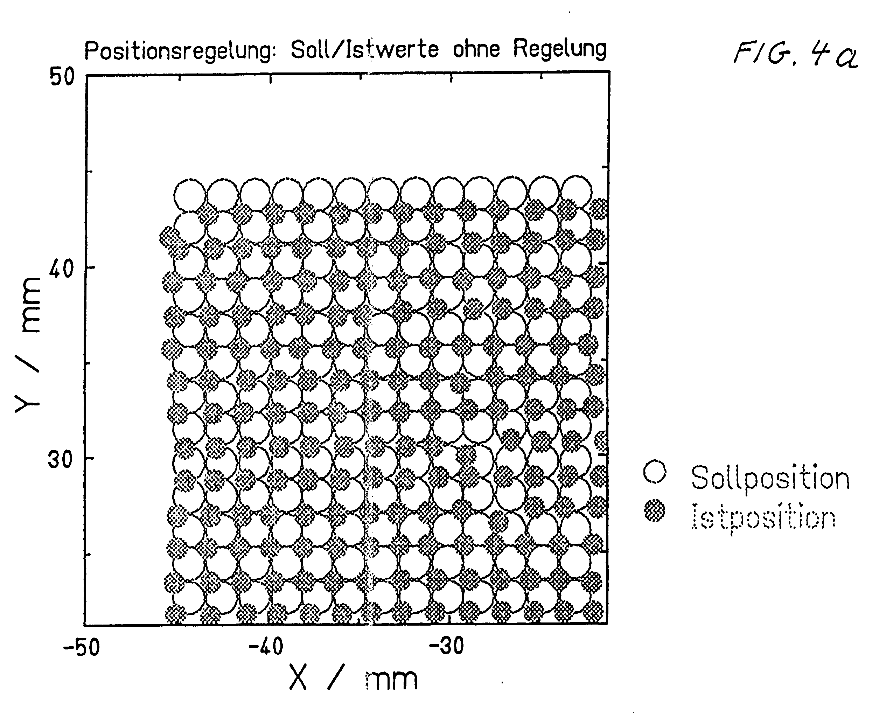

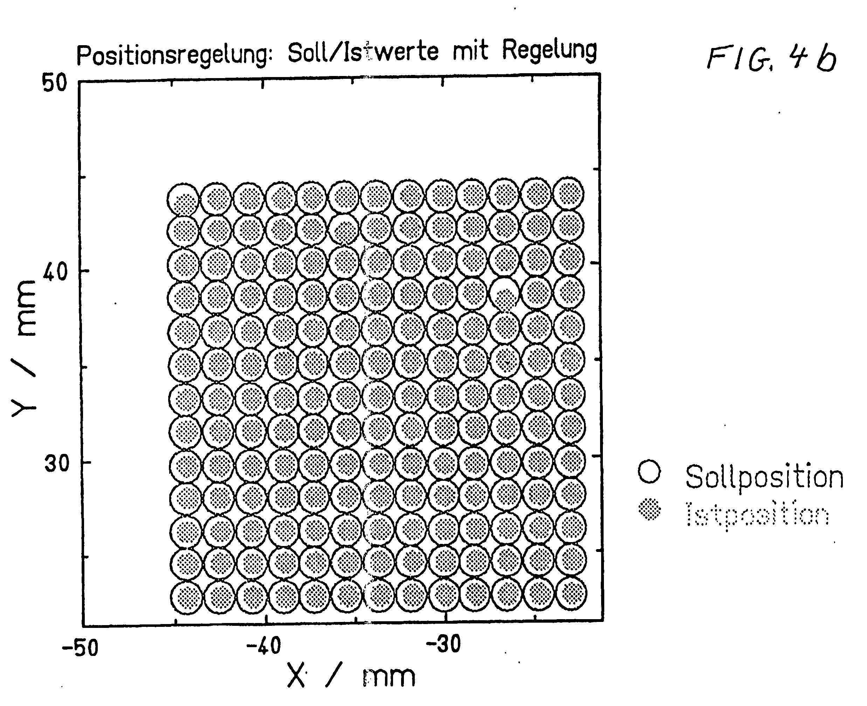

Durch die serielle Nachordnung der Steuer- und Auslesemodule (SAMS) für die horizontale (X) und vertikale (Y) Ablenkung der Steuermagnete kann vorteilhafterweise eine Echtzeitnachregelungsschleife für eine Online-Positionskontrolle und Korrektur einer jeden Strahlposition gebildet werden. Bei einer Abweichung der Istposition in einer einzelnen Strahlposition von 1.156 Positionen, wie sie im Falle der Abbildungen 3a und 3b vorliegen, oder bei der Abweichung einer einzelnen Istposition von einer Sollposition von den 2.116 geplanten Strahlpositionen der Figuren 4a und 4b greift die Regelungsschleife gemäß dem Datenflußplan der Fig. 1 ein und korrigiert bei einer Echtzeitnachregelung von Strahlposition zu Strahlposition die nächstfolgende Strahlposition, wie es mit Fig. 3b dargestellt wird.

Bei einer Echtzeitnachregelung von Meßzyklus zu Meßzyklus korrigiert und regelt die Echtzeit-Nachregelungsschleife gemäß dem Datenflußplan nach Fig. 1 den Ionenstrahl in seiner Position noch innerhalb der Dauer einer Strahlposition, da die Meßzyklusdauer kürzer ist als eine Strahlpositionsdauer. Damit ergibt sich eine Nachführung der Istposition in die Sollposition eines Behandlungsplanes für jede Strahlposition unmittelbar, so daß eine vollständige Übereinstimmung von Istwerten zu Sollwerten entsprechend der Fig. 4b in den einzelnen, in diesem Falle 2.116 Strahlpositionen, innerhalb vorgegebener Grenzen erreicht wird.

In einer bevorzugten Ausführungsform wird als ortsempfindlicher Detektor (MWPC1) eine Vieldraht-Proportional-Kammer eingesetzt. Derartige Vieldraht-Proportional-Kammern haben den Vorteil, daß eine millimetergenaue Auflösung der Ortsposition eines Ionenstrahls in der Ionenstrahltherapie dieser Ausführungsform sowohl für Ionenstrahlen aus Protonen als auch aus schwereren Ionen möglich wird.

Der in der Fig. 1 dargestellte oberste Mikroprozessor in dem Ablaufsteuergerät (VMEAS) dient als Steuer- und Auslesemodul zur Online-Datenübertragung in den Datenspeicher (ODS) in der sich anschließenden und auf der rechten Seite der Bildhälfte der Fig. 1 abgebildeten Systemkontrolle. Dieses Steuer- und Auslesemodul (SAMD) zur Online-Datenübertragung ist mit dem Datenspeicher (ODS) für eine Online-Anzeige über den Gerätebus verbunden, der ein differentieller Datenbus zwischen den Steuer- und Auslesemodulen und deren jeweiliger Frontendelektronik ist. Der Datenspeicher (ODS) für eine Online-Anzeige liefert seine Daten entsprechend dem Datenflußplan nach Fig. 1 über den Bus der Systemkontrolle (SK) und den Systemkontrollrechner in dem Bussystem (VME) zur Verbindung von Prozessoren und Datenmodulen einerseits an das Display und andererseits an das Ethernet unter Führung des Betriebssystems (AEX) in der Systemkontrolle.

In der Ausführungsform nach Fig. 1 weist das Regelsystem in der Ionenstrahlentherapie mindestens eine Ionisationskammer (IC1) auf, die der Intensitätsmessung des Ionenstrahls dient und die Ionenstrahlteilchenzahl aufaddiert, bis die Dosis für eine Strahlposition erreicht ist, so daß dann ein Befehl an den Steuer- und Auslesemodul (SAMP) für die Pulszentrale gehen kann, die über die Pulszentralenansteuerung (PZA) die Weiterschaltung auf die nächste Strahlposition veranlaßt, die dann über die Echtzeit-Nachregelungsschleife an die Magnetstrom-Netzgeräte (MGN) des Rasterscanners weitervermittelt wird. Das Steuer- und Auslesemodul (SAMI1) ist schaltungs- und ablauftechnisch innerhalb der Ablaufsteuerung (VMEAS) vor dem Steuer- und Auslesemodul (SAMO1) des ortsempfindlichen Detektors (MWPC1) in einer bevorzugten Ausführungsform der Erfindung angeordnet.

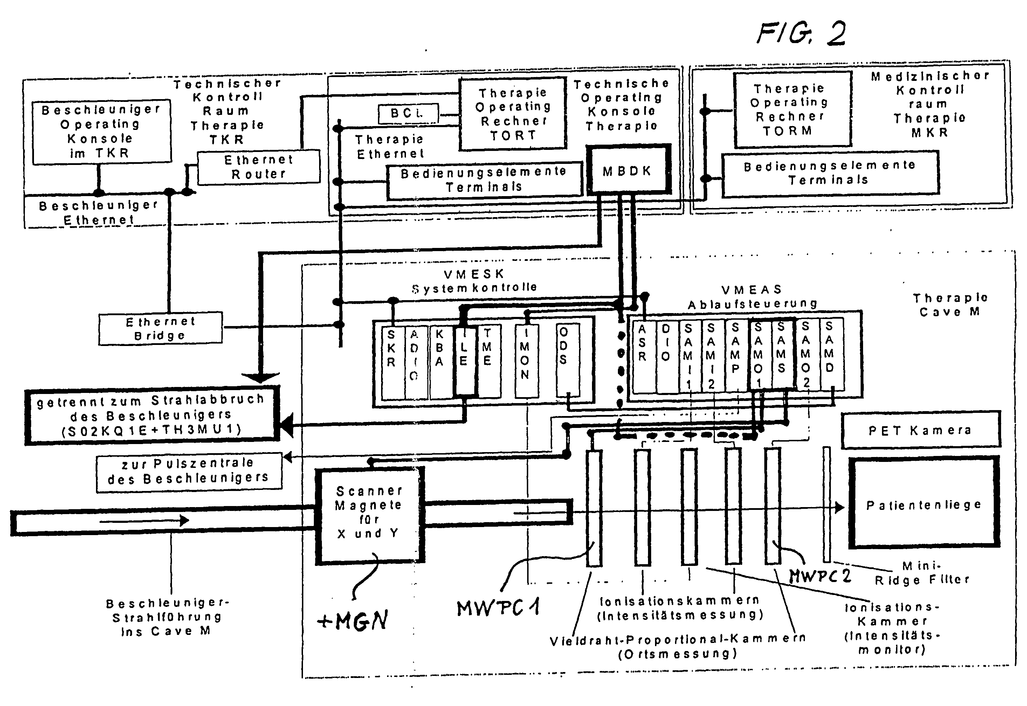

Fig. 2 zeigt eine bevorzugte Ausführungsform der Erfindung als Blockschaltbild innerhalb eines Kontrollsystems für eine Ionenstrahl-Therapieanlage. Das Kontrollsystem für eine Ionenstrahl-Therapieanlage besteht im wesentlichen aus einem technischen Kontrollraum (TKR), in dem auf einer Beschleuniger-Operating-Konsole alle Beschleunigerdaten des Ethernet auflaufen, und ein Ethernet-Router Daten an die nächste größere Einheit des Kontrollsystems für eine Ionenstrahl-Therapieanlage der technischen Operating-Konsole in der Therapie selbst weiterleitet. Das zentrale Gerät dieser technischen Operating-Konsole ist der Therapie-Operating-Rechner (TORT), der einen Barcodeleser (BCL) aufweist, und der über das Therapie-Ethernet mit dem Bedienungselement der Terminals in Verbindung steht. Die technische Operatingkonsole im Therapiebereich verfügt über eine medizinische Bedienkonsole (MBDK), die mit einem Therapiebereich (Cave M) in Verbindung steht und über eine unmittelbare Verbindung zum Auslösen eines Strahlabbruchs des Beschleunigers verfügt, wobei zum Strahlabbruch ein Resonanzquadrupol (S02KQ1E) für die langsame Extraktion des Strahls über sein Netzgerät über eine Interlock-Einheit in dem Bussystem des Therapiekontrollsystems auf Null gesetzt wird, und ein Ablenkdipolmagnet TH3MU1 der Strahlführung zum Therapiemeßplatz wird ebenfalls zum Strahl- oder Extraktionsabbruch im Fehlerfall durch die Interlock-Einheit (ILE) in dem Bussystem (VME) des Therapiekontrollsystems auf Null gesetzt wird.

Für die Systemkontrolle (VMESK) an sich wirken mehrere Mikroprozessoren auf einem Bussystem-Verbindungsrahmen (VME-CRATE) zusammen. Dazu gehört neben dem oben erwähnten und in Fig. 1 dargestellten Datenspeicher (ODS) für eine Online-Anzeige ein Intensitätsmonitor (IMON), der unter anderem mit einer Ionisationskammer und der Ausleseelektronik zur Überwachung der Gesamtteilchenzahl zusammenwirkt. Darüber hinaus befindet sich in der Systemkontrolle eine Tottmann-Schaltungseinheit (TME) zur Überwachung der Funktionsfähigkeit der Prozessoren. Neben der bereits erwähnten Interlock-Einheit (ILE) und einem Kontrollbusadapter (KBA) verfügt die Systemkonstrolle über ein Analog-Digitalmodul (ADIO) und einen Systemkontrollrechner (SKR) in dem Bussystem (VME) der Systemkontrolle.

Die Komponenten der Ablaufsteuerung (VMEAS) sind identisch mit den Komponenten des in Fig. 1 gezeigten Datenflußplans, wobei die Ablaufsteuerung in dem in Fig. 2 gezeigten Kontrollsystem zusätzlich ein digitales Eingangs-/Ausgangs-Modul (DIO) und einen Ablaufsteuerungsrechner (ASR) aufweist.

Im Therapiebereich (Cave M) befindet sich ein Positron-Emitter-Tomograph (PET) zur räumlichen Bestimmung der Teilchenreichweite durch Positronen emittierende Strahlung, mit der die Bestrahlungswirkung auf einen Patienten auf der Patientenliege nachgewiesen werden kann.

Die Ionenstrahlführung in den Therapiebereich (Cave M) hinein wird im unteren Bereich der Fig. 2 prinzipiell dargestellt, wobei der Strahl für die örtliche Abtastung durch Scannermagnete für X und Y geführt wird, die mit Hilfe von Magnetstrom-Netzgeräten (MGN) des Rasterscanners den Strahl horizontal (X) und vertikal (Y) ablenken. Nach dem Verlassen des Strahls nach dem letzten nichtgezeigten Ablenkungsmagnet wird der Strahl noch vor der Patientenliege durch eine Mehrzahl von Detektoren geführt, wobei eine Regelungsschleife über einen ersten ortsempfindlichen Detektor (MWPC1) auf die Magnetstrom-Netzgeräte (MGN) des Rasterscanners für dessen Scannermagnete wirkt, so daß die Strahlposition durch Nachführen von Strahlposition zu Strahlposition korrigiert werden kann, oder durch Nachführen von Meßzyklus zu Meßzyklus noch innerhalb einer einzelnen Strahlposition korrigiert werden kann.

Fig. 3a zeigt dazu einen Soll-/Istpositionsvergleich eines Ionen-Therapiestrahls eines Rasterscanners vor einem Einschalten einer erfindungsgemäßen Vorrichtung, wobei in dieser Darstellung auf der Ordinate Y die Position in Millimetern angegeben wird und auf der Abszisse X die Position ebenfalls in Millimetern gezeigt ist. Der Bezugspunkt für diese Positionsangaben ist das Isozentrum im Koordinatenursprung, der in diesem Fall außerhalb der dargestellten 49 Strahlpositionen liegt, wobei ein zusätzlicher ortsauflösender Detektor (MWPC2) die Übereinstimmung zwischen Soll- und Istwert im Isozentrum, also im Koordinatenursprung überwacht.

Damit kann davon ausgegangen werden, daß die Soll-Istwerte im Isozentrum bis zu einem vorgegebenen Grenzwert übereinstimmen. Jedoch weit außerhalb des Isozentrums, wie hier gezeigt, weichen die Istwerte erheblich von den Sollwerten in dem Randbereich der geplanten Bestrahlung ab. Um diese Abweichungen im Stand der Technik zu meistern, werden bisher enorm umfangreiche Korrekturtabellen durch Messen und Nachstellen erstellt, gespeichert und bei einer Bestrahlung jeweils abgefragt. Bei einer medizinischen Strahlungsanforderung von 255 unterschiedlichen Energiestufen mit jeweils 7 Fokussierstufen und jeweils 15 Intensitätsschritten ergeben sich allein daraus 25.000 Kombinationen pro Strahlposition. Verteilt auf die in dem Beispiel der Fig. 3a angegebenen 1.156 Strahlpositionen ergibt das einen Datensatz von mehr als 25.000.000. Wird die Strahlposition verdichtet, wie im Beispiel der Fig. 4a auf die dort abgebildeten 2.116 Strahlpositionen, so verdoppelt sich noch einmal diese Zahl auf über 50.000.000. Dieser nach dem Stand der Technik zu betreibende enorme Aufwand, um diese Positionsschwankungen für alle denkbaren Fälle zu vermessen und Korrekturtabellen anzulegen, welche zur Erzeugung der Steuerdaten des Systems herangezogen werden können, wird durch die erfindungsgemäße Vorrichtung und das erfindungsgemäße Verfahren zur Regelung eines Rasterscanners in der Ionenstrahlentherapie vermieden. Zumal der eben genannte und quantifizierte Aufwand im Stand der Technik, über Korrekturtabellen die Steuerung vorzunehmen, nur dann zum Ziel führt, wenn die Abweichungen reproduzierbar sind. Diese Annahme gilt jedoch nicht generell, insbesondere nicht im Hinblick auf den Einsatz der Rasterscantechnik in Kombination mit einer drehbaren Strahlführung, wie einer Gantry.

Fig. 3b zeigt einen Soll-/Istpositionsvergleich der Fig. 3a nach dem Einschalten der Vorrichtung zur Positionsregelung von Strahlposition zu Strahlposition gemäß einer Ausführungsform der Erfindung. Wie in dem Ergebnis durch Vergleich der Darstellung in Fig. 3a und in Fig. 3b zu sehen ist, werden nach Feststellung der Abweichung der Istposition von der Sollposition in der am weitesten vom Isozentrum entfernten Strahlposition in der linken oberen Ecke für die nächsten fünf Strahlpositionen eine hervorragende Übereinstimmung zwischen Istposition und Sollposition erreicht. Erst die sechste Strahlposition in der obersten Reihe weicht wieder erheblich in der Istposition von der Sollposition ab, insbesondere zeigt dann die dritte Zeile zwar gegenüber 3a verbesserte Istpositionen gegenüber der Sollposition, aber dennoch deutlich sichtbare Überschreitungen der geplanten Sollposition. In der vierten Zeile ergeben sich bei der Strahlposition-zu-Strahlposition-Regelung sehr günstige Übereinstimmungen zwischen Istposition und Sollposition, während in den weiteren noch abgebildeten drei Zeilen lediglich eine erhebliche Verbesserung gegenüber der Darstellung in 3a erreicht wird, jedoch nur selten eine kongruente Übereinstimmung zwischen Isoposition und Sollposition erreicht wird. Insgesamt wird durch Nachregelung oder Nachführung des Ionentherapiestrahls in seiner Position von geplanter Sollposition zu geplanter Sollposition gegenüber der nur im Isozentrum abgeglichenen Steuerung der Fig. 3a eine erhebliche Verbesserung mit Fig. 3b erreicht.

Fig. 4a zeigt einen weiteren Soll-/Istpositionsvergleich eines Ionen-Therapiestrahls vor einem Einschalten einer erfindungsgemäßen Vorrichtung zur Regelung eines Rasterscanners. Die Ordinate in vertikaler Y-Richtung und die Abszisse in horizontaler X-Richtung sind wieder in Millimetern vom Isozentrum bemaßt. Wie deutlich zu sehen ist, wird eine größere Fläche von 90 x 90 mm gegenüber der Darstellung in Fig. 3a mit 75 x 75 mm abgetastet und zusätzlich ist die Strahlpositionsdichte gegenüber der Darstellung in Fig. 2A wesentlich erhöht, so daß insgesamt mit 2.116 Strahlpositionen fast eine Strahlpositionsverdopplung vorliegt. Auch bei dieser Darstellung wird davon ausgegangen, daß ohne die erfindungsgemäße Regelung die Strahlposition im Isozentrum in den vorgegebenen und zulässigen Bereichen für die Istposition abgeglichen ist. Wie deutlich zu erkennen ist, ergeben sich für die Strahlpositionen in dem äußerst linken oberen Bereich vom Isozentrum aus gesehen erheblich Abweichungen zwischen Istposition und Sollposition ohne Anwendung der erfindungsgemäßen Positionsregelung. Wie oben bereits erwähnt, würde sich aufgrund der Verdopplung der Strahlpositionen der Aufwand zum Vermessen und zum Erstellen von Korrekturtabellen gegenüber der Fig. 3a bei herkömmlicher Technik verdoppeln. Durch die erfindungsgemäße Regelung des Rasterscanners kann dieser Aufwand erheblich vermindert werden.

Fig. 4b zeigt dafür einen Soll-/Istpositionsvergleich der Fig. 4a nach Einschalten der Vorrichtung zur Positionsregelung des Ionentherapiestrahls von Meßzyklus zu Meßzyklus gemäß einer weiteren Ausführungsform der Erfindung. Da die Meßzyklendauer wesentlich gegenüber der Dauer, für die ein Ionenstrahl in einer Strahlposition gehalten werden muß, um eine vorberechnete Dosis in dieser Position auf ein krankes Tumorgewebe einwirken zu lassen, verkürzt werden kann, sind mehrere Regelungszyklen zur Nachführung des Ionenstrahls in einer Strahlposition möglich, so daß die Istposition sehr genau in Übereinstimmung mit der Sollposition während der Bestrahlungsdauer einer Strahlposition in Übereinstimmung gebracht werden kann. Dieses wird mit dem Ergebnis, das sich in der Fig. 4b wiederspiegelt, deutlich.

Dazu ist die Millimetereinteilung auf der Ordinate für die vertikale Ablenkung in Y-Richtung und für die horizontale Ablenkung in X-Richtung identisch zu der Darstellung in Fig. 4a und ebenfalls die Anzahl und die Position der gezeigten Sollpositionen für die Strahlposition. Die Istposition des Strahles liegen aufgrund der Nachführung von Meßzyklus zu Meßzyklus vollständig innerhalb der geplanten Sollpositionen. Dazu errechnet die Echtzeit-Software im Steuer- und Auslesemodul (SAMO1) für den ortsempfindlichen Detektor für jeden Meßzyklus den Istwert der Strahlposition aus den Detektorrohdaten und verschickt diese Information über die Datenverbindung der beiden Steuer- und Auslesemodule an das Steuer- und Auslesemodul (SAMS) für die Strahlmagnete bzw. die Magnetstrom-Netzgeräte (MGN). Für jeden Regelzyklus, der entweder ein Meßzyklus der Ortsmessung oder ein Zyklus von Strahlposition zu Strahlposition sein kann, vergleicht die Echtzeit-Software im Steuer- und Auslesemodul (SAMS) für die Scannermagnete nun Soll- und Istposition und errechnet Stromkorrekturwerte für das horizontale und vertikale Magnetstrom-Netzgerät (MGN) des Rasterscanners und stellt dann die korrigierten Stromwerte ein, welche zur verbesserten Magnetfeldeinstellung in den Scannermagneten führen und hierdurch die Strahlposition verbessern.

Zur Vermeidung von Regelschwingungen kann die Nachführung der Strahlposition gedämpft erfolgt, weiterhin können obere Schwellen festgelegt werden, um die Korrektur zu limitieren, was bei einer derart sicherheitsrelevanten Anwendung, wie einer Ionenstrahlentherapie, äußerst wünschenswert ist. Um die Verbesserung des Ergebnisses der Fig. 4b gegenüber dem in Fig. 3b gezeigten Ergebnis zu erreichen, kann die Dauer eines Meßzyklusses um eine bis zu zwei Größenordnungen kleiner sein als die Bestrahlungsdauer einer Strahlposition, wodurch die Regelgenauigkeit erhöht werden kann, da auch höherfrequente Strahlpositionsschwankungen noch kompensiert werden können.

## Patentansprüche

1. Vorrichtung zur Regelung eines Rasterscanners in der Ionenstrahlentherapie, die mindestens folgende Geräte aufweist:
Scannermagnetstrom-Netzgeräte (MGN) für horizontal (X) und vertikal (Y) in bezug auf die Ionenstrahlmitte ablenkende Ionenstrahl-Scannermagnete, wobei die Netzgeräte (MGN) von Steuer- und Auslesemodulen (SAMS) für die Scannermagnete gesteuert werden,
einen ortsempfindlichen Detektor (MWPC1) zur Ortsmessung, der über ein Steuer- und Auslesemodul (SAMO1) gesteuert wird,
ein Ablaufsteuergerät (VMEAS), das den Ansteuerungs- und Ausleseablauf unter den Geräten der Vorrichtung steuert,
**dadurch gekennzeichnet, daß** die Vorrichtung
eine Schaltungsanordung in dem Ablaufsteuergerät (VMEAS) mit einer Rückkopplungsschleife zwischen den Steuer- und Auslesemodulen (SAMS) für die Scannermagnete und dem Steuer- und Auslesemodul (SAMO1) des ortsempfindlichen Detektors (MWPC1) aufweist, und
schaltungs- und ablauftechnisch die Steuer- und Auslesemodule (SAMS) für die Scannermagnete und das Steuer- und Auslesemodul (SAMO1) des ortsempfindlichen Detektors (MWPC1) in dem Ablaufsteuergerät (VMEAS) derart angeordnet sind, daß die Steuer- und Auslesemodule (SAMS) für die Scannermagnete dem Steuer- und Auslesemodul (SAMO1) des ortsempfindlichen Detektors (MWPC1) seriell nachgeordnet sind.

2. Vorrichtung nach Anspruch 1, **dadurch gekennzeichnet, daß** als ortsempfindlicher Detektor (MWPC1) eine Vieldraht-Proportional-Kammer eingesetzt ist.

3. Vorrichtung nach Anspruch 1 oder 2, **dadurch gekennzeichnet, daß** sie mindestens eine Ionisationskammer (IC1) zur Intensitätsmessung des Ionenstrahls aufweist, deren Steuer- und Auslesemodul (SAMI1) innerhalb der Ablaufsteuerung (VMEAS) schaltungs- und ablauftechnisch vor dem Steuer- und Auslesemodul (SAMO1) des ortsempfindlichen Detektors (MWPC1) angeordnet ist.

4. Verfahren zur Regelung eines Rasterscanners in der Ionenstrahlentherapie, wobei der Rasterscanner mindestens folgende Geräte aufweist:
Scannermagnetstrom-Netzgeräte (MGN) für horizontal (X) und vertikal (Y) in bezug auf die Ionenstrahlmitte ablenkende Ionenstrahl-Scannermagnete, wobei die Netzgeräte (X, Y) von Steuer- und Auslesemodulen (SAMS) für die Scannermagnete gesteuert werden,
einen ortsempfindlichen Detektor (MWPC1) zur Ortsmessung, der über ein Steuer- und Auslesemodul (SAMO1) gesteuert wird,
ein Ablaufsteuergerät (VMEAS), das den Ansteuerungs- und Ausleseablauf unter den Geräten der Vorrichtung steuert, und
das Verfahren durch folgende Schritte **gekennzeichnet ist**:
Vergleichen einer in dem Ortsmeßsteuer- und Auslesemodul (SAMO1) abgelegten Information über die Sollposition der Bestrahlungsplanung mit der gemessenen Istposition der Strahlposition aus dem ortsempfindlichen Detektor (MWPC1) in Echtzeit.
Ermitteln eines Korrekturwertes für die Scannermagnetstrom-Netzgeräte (MGN) des Rasterscanners, und
Einstellen des Korrekturwertes für die horizontalen und vertikalen Scannermagnetstrom-Netzgeräte (X, Y) des Rasterscanners und Nachführen der Strahlposition.

5. Verfahren nach Anspruch 4, **dadurch gekennzeichnet, daß** das Nachführen von Strahlposition zu Strahlposition durchgeführt wird.

6. Verfahren nach Anspruch 4, **dadurch gekennzeichnet, daß** das Nachführen von Meßzyklus zu Meßzyklus durchgeführt wird, wobei die Meßzyklusdauer kürzer ist als eine Strahlpositionsdauer.

7. Verfahren nach einem der Ansprüche 4 bis 6, **dadurch gekennzeichnet, daß** für jeden Meßzyklus eine Echtzeit-Software in dem Steuer- und Auslesemodul (SAMO1) des ortsempfindlichen Detektors (MWPC1) den Istwert der Strahlposition aus den Detektordaten errechnet und diese Information über eine Datenverbindung zwischen dem Steuer- und Auslesemodul (SAMO1) des ortsempfindlichen Detektors (MWPC1) und den Steuer- und Auslesemodulen(SAMS) für die Scannermagnete verschickt.

8. Verfahren nach einem der Ansprüche 4 bis 7, **dadurch gekennzeichnet, daß** für einen Regelzyklus eine Echtzeit-Software, die Soll- und Istposition der Strahlposition vergleicht und Stromkorrekturwerte für das horizontale und das vertikale Scannermagnetstrom-Netzgerät (MGN) des Rasterscanners errechnet und die korrigierten Stromwerte einstellt.

9. Verfahren nach einem der Ansprüche 4 bis 8, **dadurch gekennzeichnet, daß** die Strahlnachführung mit einer über eine Echtzeit-Software in den Steuer- und Auslesemodulen für die Scannermagnete (SAMS) einstellbaren Dämpfung durchgeführt wird.

10. Verfahren nach einem der Ansprüche 4 bis 9, **dadurch gekennzeichnet, daß** eine schnelle Strahlabschaltung von dem Steuer- und Auslesemodul (SAMO1) des ortsempfindlichen Detektors in Echtzeit ausgelöst wird, falls die Differenz zwischen dem Meßwert und Sollwert der Strahlposition eine in einer Echtzeit-Software des Steuer- und Auslesemoduls (SAMO1) des ortsempfindlichen Detektors (MWPC1) einstellbare Schwelle übersteigt.

## Claims

1. Apparatus for the feedback control of a grid scanner in ion beam therapy, which has, at least, the following devices:
scanner magnet current supply devices (MGN) for ion beam scanner magnets that deflect horizontally (X) and vertically (Y) with respect to the middle of the ion beam, the supply devices (MGN) being controlled by control and read-out modules (SAMS) for the scanner magnets,
a location-sensitive detector (MWPC1) for location measurement, which is controlled by means of a control and read-out module (SAMO1),
a sequence control device (VMEAS), which controls the activation and read-out sequence among the devices of the apparatus,
**characterized in that** the apparatus
has in the sequence control device (VMEAS) a circuit arrangement having a feedback loop between the control and read-out modules (SAMS) for the scanner magnets and the control and read-out module (SAMO1) of the location-sensitive detector (MWPC1) and,
in circuitry and sequence, the control and read-out modules (SAMS) for the scanner magnets and the control and read-out module (SAMO1) of the location-sensitive detector (MWPC1) in the sequence control device (VMEAS) are technically so arranged that the control and read-out modules (SAMS) for the scanner magnets are arranged serially after the control and read-out module (SAMO1) of the location-sensitive detector (MWPC1).

2. Apparatus according to claim 1, **characterized in that** a multi-wire proportional chamber is used as the location-sensitive detector (MWPC1).

3. Apparatus according to claim 1 or 2, **characterized in that** it has at least one ionisation chamber (IC1) for measuring the intensity of the ion beam, the control and read-out module (SAMI1) of which ionisation chamber is, within the sequence control (VMEAS), technically arranged, in circuitry and sequence, before the control and read-out module (SAMO1) of the location-sensitive detector (MWPC1).

4. Method for the feedback control of a grid scanner in ion beam therapy, wherein the grid scanner has, at least, the following devices:
scanner magnet current supply devices (MGN) for ion beam scanner magnets that deflect horizontally (X) and vertically (Y) with respect to the middle of the ion beam, the supply devices (X, Y) being controlled by control and read-out modules (SAMS) for the scanner magnets,
a location-sensitive detector (MWPC1) for location measurement, which is controlled by means of a control and read-out module (SAMO1),
a sequence control device (VMEAS), which controls the activation and read-out sequence among the devices of the apparatus, and
wherein the method is **characterized by** the following steps:
comparison of information, deposited in the location measurement control and read-out module (SAMO1) and relating to the desired position of the beam plan, with the actual measured position of the beam position from the location-sensitive detector (MWPC1), in real time,
determination of a correction value for the scanner magnet current supply devices (MGN) of the grid scanner, and
setting the correction value for the horizontal and vertical scanner magnet current supply devices (X, Y) of the grid scanner and realignment of the beam position.

5. Method according to claim 4, **characterized in that** the realignment is carried out from beam position to beam position.

6. Method according to claim 4, **characterized in that** the realignment is carried out from measurement cycle to measurement cycle, the duration of a measurement cycle being shorter that the duration of a beam position.

7. Method according to any one of claims 4 to 6, **characterized in that**, for each measurement cycle, real-time software in the control and read-out module (SAMO1) of the location-sensitive detector (MWPC1) calculates the actual value of the beam position from the detector data and sends that information by way of a data connection between the control and read-out module (SAMO1) of the location-sensitive detector (MWPC1) and the control and read-out modules (SAMS) for the scanner magnets.

8. Method according to any one of claims 4 to 7, **characterized in that**, for a feedback control cycle, real-time software compares the desired position and the actual position of the beam position and calculates current-correction values for the horizontal and vertical scanner magnet current supply device (MGN) of the grid scanner and sets the corrected current values.

9. Method according to any one of claims 4 to 8, **characterized in that** beam realignment is carried out using damping that is arranged to be set by means of the real-time software in the control and read-out modules for the scanner magnets (SAMS).

10. Method according to any one of claims 4 to 9, **characterized in that** rapid switching-off of the beam is triggered by the control and trigger module (SAMO1) of the location-sensitive detector in real time if the difference between the measured value and the desired value of the beam position exceeds a threshold that is arranged to be set in the real-time software of the control and read-out module (SAMO1) of the location-sensitive detector (MWPC1).

## Revendications

1. Dispositif de réglage d'un scanner par trame en thérapie par faisceaux ioniques, qui comporte au moins les appareils suivants :
des appareils d'alimentation en courant pour l'aimant du scanner (MGN) pour l'horizontale (X) et la verticale (Y) par rapport à des aimants du scanner à faisceaux ioniques déviant le centre du faisceau ionique, les appareils d'alimentation (MGN) étant pilotés par des modules de commande et de lecture (SAMS) pour les aimants du scanner,
un détecteur sensible à la position (MWPC1) pour la mesure de position, qui est piloté par un module de commande et de lecture (SAM01),
un organe de commande du processus (VMEAS), qui pilote le processus de commande et de lecture sous les appareils du dispositif,
**caractérisé en ce que** le dispositif
comporte un agencement de circuits dans l'organe de commande du processus (VMEAS), avec une boucle de rétroaction entre les modules de commande et de lecture (SAMS) pour les aimants du scanner et le module de commande et de lecture (SAM01) du détecteur sensible à la position (MWPV) et
du point de vue de la technique du circuit et du processus, les modules de commande et de lecture (SAMS) pour les aimants du scanner et le module de commande et de lecteur (SAM01) du détecteur sensible à la position (MWPC1) sont agencés dans l'organe de commande du processus (VMEAS)de façon telle, que les modules de commande et de lecture (SAMS) pour les aimants du scanner sont montés en série en aval du module de commande et de lecture (SAM01) du détecteur sensible à la position (MPWC1).

2. Dispositif selon la revendication 1, **caractérisé en ce qu'**une chambre proportionnelle multifils est utilisée en tant que détecteur sensible à la position (MWPC1).

3. Dispositif selon la revendication 1 ou 2, **caractérisé en ce qu'**il comporte au moins une chambre d'ionisation (IC1) pour la mesure d'intensité du faisceau ionique, dont le module de commande et de lecture (SAMI1) est agencé à l'intérieur de la commande de processus (VMEAS), du point de vue technique du circuit et du processus, en amont du module de commande et de lecture (SAM01) du détecteur sensible à la position (MWPC1).

4. Procédé pour le réglage d'un scanner par trame en thérapie par faisceaux ioniques, le scanner comportant au moins les appareils suivants :
des appareils d'alimentation en courant pour l'aimant du scanner (MGN) pour l'horizontale (X) et la verticale (Y) par rapport à des aimants du scanner à faisceaux ioniques déviant le centre du faisceau ionique, les appareils d'alimentation (X, Y) étant pilotés par des modules de commande et de lecture (SAMS) pour les aimants du scanner,
un détecteur sensible à la position (MWPC1) pour la mesure de position, qui est piloté par un module de commande et de lecture (SAM01),
un organe de commande du processus (VMEAS), qui pilote le processus de commande et de lecture sous les appareils du dispositif,
le procédé étant **caractérisé par** les étapes suivantes :
comparaison d'une information sauvegardée dans le module de mesure de position et de lecture (SAM01), à l'aide de la position de consigne du plan d'irradiation avec la position réelle du faisceau mesurée à partir du détecteur sensible à la position (MWPC1) en temps réel,
recherche d'une valeur de correction pour les appareils d'alimentation en courant pour l'aimant du scanner (MGN) du scanner par trame et
réglage de la valeur de correction pour les appareils horizontaux et verticaux (X, Y) d'alimentation en courant pour l'aimant du scanner (MGN) du scanner par trame et poursuite de la position du faisceau.

5. Procédé selon la revendication 4, **caractérisé en ce que** la poursuite est réalisée de position du faisceau en position du faisceau.

6. Procédé selon la revendication 4, **caractérisé en ce que** la poursuite est réalisée de cycle de mesure en cycle de mesure, la durée du cycle de mesure étant inférieure à une durée de position du faisceau.

7. Procédé selon l'une quelconque des revendications 4 à 6, **caractérisé en ce que** pour chaque cycle de mesure, un logiciel en temps réel dans le module de commande et de lecture (SAM01) du détecteur sensible à la position (MWPC1) calcule la valeur réelle de la position du faisceau à partir des données du détecteur et envoie cette information par l'intermédiaire d'une liaison de données entre le module de commande et de lecture (SAM01) du détecteur sensible à la position (MWPC1) et les modules de commande et de lecture (SAMS) pour les aimants du scanner.

8. Procédé selon l'une quelconque des revendications 4 à 7, **caractérisé en ce que** pour un cycle de réglage, un logiciel en temps réel compare la position de consigne et la position réelle du faisceau et calcule des valeurs de correction du courant pour l'appareil horizontal et vertical d'alimentation en courant pour l'aimant du scanner (MGN) du scanner par trame et règle les valeurs corrigées.

9. Procédé selon l'une quelconque des revendications 4 à 8, **caractérisé en ce que** la poursuite du faisceau est réalisée à l'aide d'un logiciel en temps réel dans les modules de commande et de lecture pour l'amortissement réglable des aimants du scanner (SAMS).

10. Procédé selon l'une quelconque des revendications 4 à 9, **caractérisé en ce qu'**une coupure rapide du faisceau est déclenchée en temps réel par le module de commande et de lecture (SAM01) du détecteur sensible à la position, si la différence entre la valeur mesurée et la valeur de consigne pour la position du faisceau dépasse un seuil réglable dans un logiciel en temps réel du module de commande et de lecture (SAM01) du détecteur sensible à la position (MWPC1).
